(19) Europäisches Patentamt
European Patent Office
Office européen des brevets

(11) Veröffentlichungsnummer: **0 000 479**
**B1**

(12) # EUROPÄISCHE PATENTSCHRIFT

(45) Veröffentlichungstag der Patentschrift: **07.01.81**

(21) Anmeldenummer: **78100263.9**

(22) Anmeldetag: **28.06.78**

(51) Int. Cl.³: **C 07 D 495/14,** A 61 K 31/55 // (C07D495/14, 333/00, 249/00, 243/00)

(54) **Substituierte 1-Piperazinyl-4H-s-triazolo (3,4-c)thieno(2,3-e)1,4-diazepine, Verfahren zu deren Herstellung und diese enthaltende Arzneimittel.**

(30) Priorität: **21.07.77 DE 2732943**
**21.07.77 DE 2732921**

(43) Veröffentlichungstag der Anmeldung: **07.02.79 Patentblatt 79/3**

(45) Bekanntmachung des Hinweises auf die Patenterteilung: **07.01.81 Patentblatt 81/1**

(84) Benannte Vertragsstaaten: **DE NL**

(56) Entgegenhaltungen:
**FR-A-2 262 525**
**US-A-3 894 025**

(73) Patentinhaber: **C.H. BOEHRINGER SOHN, Postfach 200, D-6507 Ingelheim am Rhein (DE)**

(72) Erfinder: **Weber, Karl-Heinz, Dr., Kaiser-Karl-Strasse 11, D-6535 Gau-Algesheim (DE)**
Erfinder: **Langbein, Adolf, Dr., Am Goldberg 6, D-6535 Gau-Algesheim (DE)**
Erfinder: **Schneider, Claus, Dr., Tannenweg 1, D-6507 Ingelheim/Rhein (DE)**
Erfinder: **Lehr, Erich, Dr., In der Toffel 5, D-6531 Waldalgesheim (DE)**
Erfinder: **Böke, Karin, Dr., Selztalstrasse 99, D-6507 Ingelheim/Rhein (DE)**
Erfinder: **Kuhn, Franz Josef, Dr., Melchiorstrasse 6, D-6530 Bingen 13 (DE)**

**EP 0 000 479 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# Substituierte 1-Piperazinyl-4H-s-triazolo [3,4-c]thieno[2,3-e]1,4-diazepine, Verfahren zu deren Herstellung und diese enthaltende Arzneimittel

Die Erfindung betrifft neue substituierte 1-Piperazinyl-4H-s-triazolo[3,4c]thieno[2,3e]1,4-diazepine der allgemeinen Formel

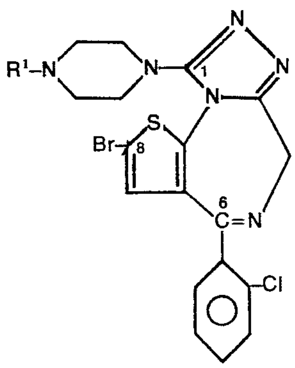

(I)

in der
$R^1$ die 2-Hydroxyäthylgruppe oder den 2-Pyridylrest bedeutet, Verfahren zu ihrer Herstellung und ihre Verwendung als Wirkstoffe in Arzneimitteln.

Thieno-triazolo-diazepine sind als anxiolytisch, muskelrelaxierend und antikonvulsiv wirkend bekannt. Es handelt sich hierbei um solche Verbindungen, die am Triazoloring beispielsweise durch Alkyl (DT-OS 2405682), Cycloalkyl (DBP 2435041), einen Sauerstoff enthaltenden 5- oder 6gliedrigen Ring (DT-AS 2445430) oder einen Stickstoff oder Schwefel enthaltenden 5- oder 6gliedrigen Ring (DT-OS 2460776) substituiert sind.

Die neuen Verbindungen weisen gegenüber den bekannten Stoffen verschiedene Vorteile auf:

So kann das 8-Brom-6-(o-chlorphenyl)-1-[N-(2-hydroxyäthyl)-piperazinyl]-4H-s-triazolo [3,4c]thieno[2,3e]1,4-diazepin Salze bilden und besitzt in dieser Form eine für die Substanzklasse der Diazepine bedeutende Wasserlöslichkeit von 0,5%. Diese gestattet die parenterale Anwendung der Verbindung, z.B. in der Narkosevorbereitung.

8-Brom-6-(o-chlorphenyl)-1-[N-(2-pyridyl)-piperazinyl]-4H-s-triazolo[3,4c]thieno[2,3e]1,4-diazepin bildet keine Salze; im Gegensatz zu den bekannten Benzodiazepinen wird das Schlaf-Wach-Verhalten der Katze global wenig beeinflusst; Tief- und REMschlaflatenz werden nicht verändert. Eine ausgeprägte Schlafinduktion ist nicht gegeben, der REMschlafanteil wird nicht vermindert. Im Gegensatz zu 8-Brom-6-(o-chlorphenyl)-1-[N-(2-pyridyl)-piperazinyl]-4H-s-triazolo[3,4c]thieno[2,3e]1,4-diazepin verlängert 8-Brom-6-(o-chlorphenyl)-1-methyl-4H-s-triazolo[3,4c]thieno[2,3e]1,4-diazepin den Tief- und den REMschlaf und beeindruckt in wesentlich stärkerem Mass die Motorkoordination.

Man kann die beiden Verbindungen erhalten

a) durch Umsetzung eines Triazolo-thieno-diazepins der allgemeinen Formel

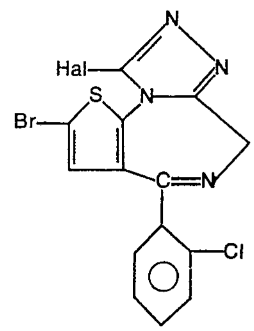

(II)

in der
Hal ein Halogenatom bedeutet, mit einem Piperazin der Formel

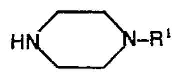

(III)

in der
$R^1$ die oben angegebene Bedeutung hat, oder

b) durch Dehydrierung einer Verbindung der allgemeinen Formel

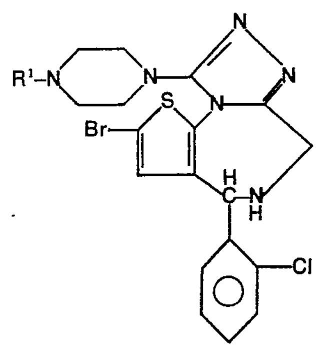

(IV)

in der
$R^1$ die oben angegebene Bedeutung hat.

c) Die 2-Hydroxyäthylpiperazinylverbindung ist ausserdem herstellbar durch Umsetzung des 8-Brom-6-(o-chlorphenyl)-1-piperazinyl-4H-s-triazolo[3,4c]thieno[2,3e]-1,4-diazepins mit Äthylenoxyd.

Die Umsetzung von Verbindungen der allgemeinen Formel II mit einem Piperazin der Formel III erfolgt entweder ohne Lösungsmittel oder in höher siedenden Lösungsmitteln wie Benzol, Toluol, Dioxan, Tetrahydrofuran, Chlorkohlenwasserstoffen wie Tetrachlorkohlenstoff oder Methylenchlorid, vorzugsweise bei der Siedetemperatur des jeweils verwendeten Lösungsmittels. Die Reaktionsdauer ist abhängig vom eingesetzten Ausgangsmaterial und kann wenige Minuten bis mehrere Stunden betragen.

Die Dehydrierung von Verbindungen der allgemeinen Formel IV erfolgt unter Verwendung geeigneter Dehydrierungsmittel wie z.B. von Halogenen oder auch von Verbindungen der höheren Oxydationsstufen des Chroms oder Mangans,

beispielsweise eines Chromats, eines Bichromats oder eines Permanganats.

Als geeignete Lösungsmittel für die Umsetzung mit einem Halogen seien Chlorkohlenwasserstoffe wie Chloroform oder Methylenchlorid genannt. Die Oxydation mit den erwähnten Verbindungen des Chroms oder Mangans erfolgt in Lösungsmitteln wie Aceton, Tetrahydrofuran oder Dioxan. Je nach Art des Oxydationsmittels liegt die Reaktionstemperatur im allgemeinen zwischen 0°C und der Siedetemperatur des verwendeten Lösungsmittels.

Das 8-Brom-6-(o-chlorphenyl)-1-[N-(2-hydroxyäthyl)-piperazinyl]-4H-s-triazolo[3,4c]thieno no[2,3e]1,4-diazepin bildet stabile wasserlösliche Salze. Zur Salzbildung geeignet sind alle Säuren, die physiologisch verträgliche Säureadditionssalze bilden wie Halogenwasserstoffsäuren, Schwefelsäure, Phosphorsäure, Salpetersäure, Cyclohexylsulfaminsäure, Zitronensäure, Weinsäure, Ascorbinsäure, Maleinsäure, Ameisensäure, Salicylsäure, Methan- oder Toluolsulfonsäure.

Die Ausgangsverbindungen der allgemeinen Formel II sind literaturbekannt. Ausgangsverbindungen der allgemeinen Formel IV erhält man durch Umsetzung einer Verbindung der Formel

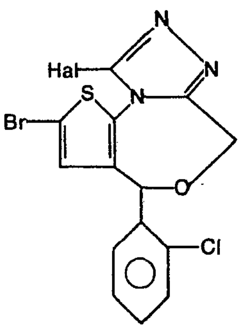

(VI)

in der

Hal ein Halogenatom bedeutet, mit einem Piperazinderivat der Formel III unter den bei a) angegebenen Bedingungen und anschliessendem Austausch des Ring-Sauerstoff-Atoms durch ein Stickstoffatom, wie er z.B. in der deutschen Anmeldung P 25 31 678 beschrieben worden ist.

Die Einzeldosis der erfindungsgemässen Substanzen liegt bei 0,05 bis 50, vorzugsweise 0,1 bis 25 mg (oral) und 5 bis 150 mg als Tagesdosis.

Die erfindungsgemäss erhältlichen Verbindungen können allein oder in Kombination mit anderen erfindungsgemässen Wirkstoffen, gegebenenfalls auch in Kombination mit weiteren pharmkologisch aktiven Wirkstoffen wie Spasmolytica oder β-Rezeptorenblocker zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemässen Wirkstoffe beziehungsweise Wirkstoffkombinationen können zusätzlich noch ein Süssungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können ausserdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Beispiel 1

8-Brom-6-(o-chlorphenyl)-1-[N-(2-hydroxyäthyl)-piperazinyl]-4H-s-triazolo[3,4c]thieno[2,3e]1,4-diazepin

0,07 Mol = 31 g 1,8-Dibrom-6-(O-chlorphenyl)-4H-s-triazolo-[3,4c]thieno[2,3e]1,4-diazepin, 17 g (0,014 Mol) N-(2-Hydroxyäthyl)-piperazin und 800 ml Xylol werden 24 Stunden unter Rückfluss gekocht. Man saugt das Reaktionsgemisch noch warm über Kieselgur-Kohle ab und dampft das Filtrat im Vakum ein. Der Rückstand wird in Methylenchlorid aufgenommen und die Lösung mit Wasser gewaschen. Nach dem Trocknen und Eindampfen erhält man 28,5 g = 81% d.Th. der Titelverbindung vom Fp. 125–126°C (aus Essigester).

Die Base wird mit wenig Alkohol suspendiert und mit einem kleinen Überschuss alkoholischer Salzsäure versetzt. Nach Zugabe von Äther kristallisiert das gut wasserlösliche Hydrochlorid der Titelverbindung, Fp. 211–220°C (Zers.).

In analoger Weise erhält man aus der in heissem Alkohol gelösten Base durch Zusatz von 1 Mol Maleinsäure, Weinsäure beziehungsweise Methansulfonsäure die entsprechenden Salze in quantitativer Ausbeute.
Maleat: Fp. 201–202°C
Tartrat: Fp. 228–229°C
Methansulfonat: Fp. 241–242°C

Beispiel 2
8-Brom-6-(o-chlorphenyl)-1-[N-(2-pyridyl)-piperazinyl]-4H-s-triazolo[3,4c]thieno[2,3e]1,4-diazepin

0,04 Mol = 19 g 1,8-Dibrom-6-(o-chlorphenyl)-4H-s-triazolo[3,4c]thieno[2,3e]1,4-diazepin werden mit 0,08 Mol = 13,4 g N-[Pyridyl-(2)]-piperazin und 150 ml Xylol 24 Stunden unter Rückfluss gekocht. Das ausgefallene N-[pyridyl-(2)]-piperazin-Hydrobromid wird abgesaugt. Im Filtrat befindet sich die Titelverbindung, die man nach Eindampfen und Umkristallisieren aus Äthanol isoliert und reinigt.

Ausbeute: 13,2 g = 61% d.Th., Fp. 215–216°C.

Formulierungsbeispiele
a) Dragees
1 Drageekern enthält:

| | |
|---|---|
| Wirkstoff gemäss der Erfindung | 1,0 mg |
| Milchzucker | 28,5 mg |
| Maisstärke | 19,0 mg |
| Gelatine | 1,0 mg |
| Magnesiumstearat | 0,5 mg |
| | 50,0 mg |

Herstellung

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpresst. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragees werden mit Bienenwachs poliert. Dragee-Endgewicht: 100 mg

b) Tabletten

| | |
|---|---|
| Wirkstoff gemäss der Erfindung | 0,5 mg |
| Milchzucker | 50,0 mg |
| Maisstärke | 43,5 mg |
| lösliche Stärke | 5,0 mg |
| Magnesiumstearat | 1,0 mg |
| | 100,0 mg |

Herstellung:

Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpresst, die je 0,5 mg Wirkstoff enthalten.

c) Suppositorien
1 Zäpfchen enthält:

| | |
|---|---|
| Wirkstoff gemäss der Erfindung | 5,0 mg |
| Zäpfchenmasse | 1.695,0 mg |

Herstellung:
Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) Ampullen (Injektionslösungen)
Zusammensetzung:

| | |
|---|---|
| Wirkstoff gemäss der Erfindung | 0,5 Gew.-Teile |
| Natriumpyrosulfit | 1,0 Gew.-Teile |
| Dinatriumsalz der Äthylendiamintetraessigsäure | 0,5 Gew.-Teile |
| Natriumchlorid | 8,5 Gew.-Teile |
| doppelt destilliertes Wasser ad | 1000,0 Gew.-Teile |

Herstellung:

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 0,5 mg Wirkstoff.

**Patentansprüche**

1. Neue substituierte 1-Piperazinyl-4H-s-triazolo[3,4c]thieno[2,3e]1,4-diazepine der allgemeinen Formel

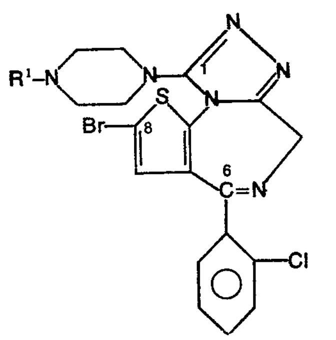

(I)

in der
$R^1$ die 2-Hydroxyäthylgruppe oder den 2-Pyridylrest bedeutet und die Säureadditionssalze der zuerst genannten Verbindung.

2. 8-Brom-6-(o-chlorphenyl)-1-[N-(hydroxyäthyl)-piperazinyl]-4H-s-triazolo[3,4c]thieno[2,3e]1,4-diazepin gemäss Anspruch 1 und dessen physiologisch verträgliche Säureadditionssalze.

3. 8-Brom-6-(o-chlorphenyl)-1-[N-(2-pyridyl)-piperazinyl]-4H-s-triazolo[3,4c]thieno[2,3e]1,4-diazepin gemäss Anspruch 1.

4. Verfahren zur Herstellung neuer substituierter 1-Piperazinyl-4H-s-triazolo[3,4c]thieno[2,3e] 1,4-diazepine der Formel

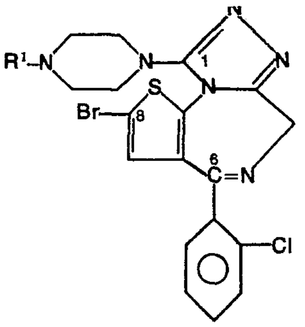

(I)

in der
$R^1$ die 2-Hydroxyäthylgruppe oder den 2-Pyridylrest bedeutet, dadurch gekennzeichnet, dass man

a) eine Verbindung der Formel

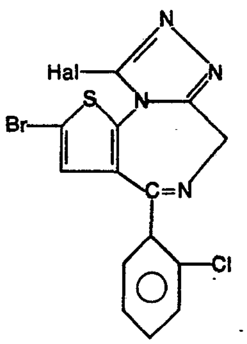

(II)

in der
Hal ein Halogenatom bedeutet, mit einem Piperazin der Formel

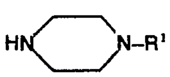

(III)

in der
$R^1$ die oben genannte Bedeutung hat, umsetzt, oder dass man

b) eine Verbindung der allgemeinen Formel

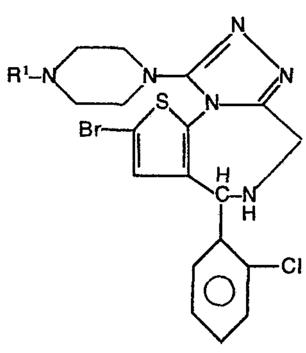

(IV)

in der
$R^1$ die oben genannte Bedeutung hat, in an sich bekannter Weise dehydriert, oder dass man

c) zur Herstellung der 1-(2-Hydroxyäthyl)-piperazinyl-Verbindung das 8-Brom-6-(o-chlorphenyl)-1-piperazinyl-4H-s-triazolo[3,4c]thieno [2,3e]1,4-diazepin mit Äthylenoxyd umsetzt, und dass man, im Falle $R^1$ die 2-Hydroxyäthylgruppe bedeutet, das so erhaltene Endprodukt der Formel I gegebenenfalls in üblicher Weise in ein physiologisch verträgliches Säureadditionssalz überführt.

5. Pharmazeutische Präparate, enthaltend als Wirkstoff Verbindungen der allgemeinen Formel I gemäss Anspruch 1, gegebenenfalls in Form ihrer Säureadditionssalze, in Kombination mit üblichen Hilfs- und/oder Trägerstoffen.

**Claims**

1. New substituted 1-piperazinyl-4H-s-triazolo-[3,4c]thieno[2,3e]1,4-diazpines of general formula

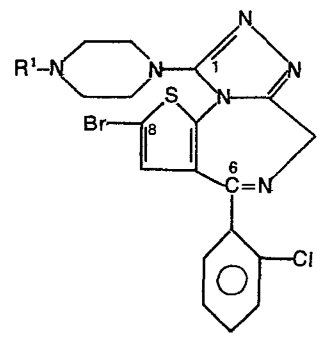

(I)

wherein
$R^1$ is hydroxy-ethyl or 2-pyridyl and a non-toxic, pharmacologically acceptable acid addition salt of the compound first mentioned.

2. 8-Bromo-6-(o-chloro-phenyl)-1-[hydroxy-ethyl)-piperazino]-4H-s-triazolo[3,4c)thieno [2,3e]1,4-diazepine according to claim 1 or a non-toxic, pharmacologically acceptable acid addition salt thereof.

3. 8-Bromo-6-(o-chloro-phenyl)-1-[N-(2-pyridyl)-piperazinyl]-4H-s-triazolo[3,4c]thieno[2,3e] 1,4-diazepine according to claim 1.

4. Processes for the production of new substituted 1-piperazinyl-4H-2-triazolo[3,4c]thieno [2,3e]1,4-diazepines of formula

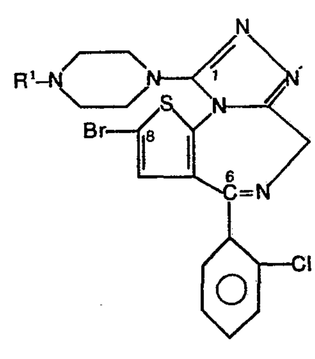

(I)

wherein R¹ is hydroxy-ethyl or 2-pyridyl comprising

a) reacting a compound of formula

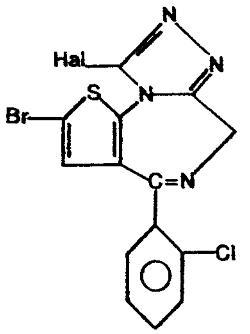

(II)

wherein Hal is halogen, with a piperazine of the formula

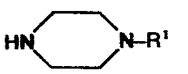

(III)

wherein R¹ has the same meanings as in formula I, or

b) de-hydrogenating a compound of general formula

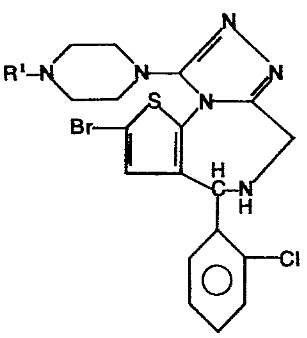

(IV)

wherein R¹ the same meanings as in formula I, or,

c) for the preparation of the 1-(2-hydroxy-ethyl)-piperazino compound, reacting 8-bromo-6-(o-chloro-phenyl)-1-piperazino-4H-s-triazolo[3,4c]thieno-[2,3e]1,4-diazepine with ethyleneoxid and in case R¹ is 2-hydroxy-ethyl, converting the end product thus obtained if desired, into a physiologically acceptable acid addition salt.

5. Pharmaceutical composition containing as active ingredient compounds of general formula I according to claim 1 if desired in form of their acid addition salts in combination with conventional excipients and/or carriers.

**Revendications**

1. Nouvelles 1-pipérazinyl-4H-s-triazolo[3,4c]thiéno[2,3e]1,4-diazépines substituées caractérisées en ce qu'elles correspondent à la formule générale:

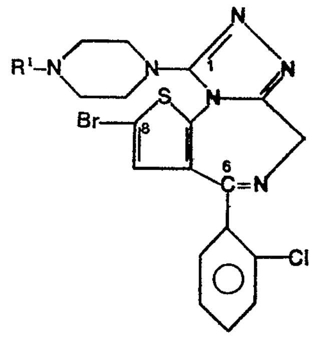

(I)

dans laquelle
R¹ signifie le radical hydroxyéthyl ou 2-pyridyle et les sels d'addition du composé mentionné d'abord.

2. 8-Bromo-6-(o-chlorophényl)-1-[N-(hydroxyéthyl)-pipérazinyl]-4H-s-triazolo[3,4c]thiéno[2,3e]1,4-diazépine selon la revendication 1 et ses sels d'addition d'acide physiologiquement compatibles.

3. 8-Bromo-6-(o-chlorophényl)-1-[N-(2-pyridyl)-pipérazinyl]-4H-s-triazolo[3,4c]thiéno[2,3e]1,4-diazépine selon la revendication 1.

4.Procédé d'obtention de nouvelles 1-pipérazinyl-4H-s-triazolo[3,4c]thiéno[2,3e]1,4-diazépines substituées de formule générale

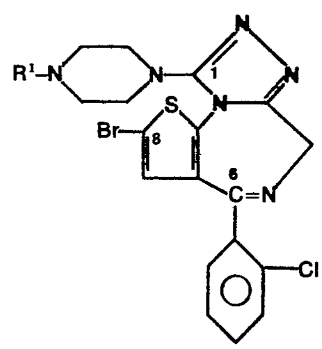

(I)

dans laquelle R¹ signifie le radical hydroxyéthyl ou 2-pyridyle, caractérisé par le fait que

a) on fait réagir un composé de formule générale II

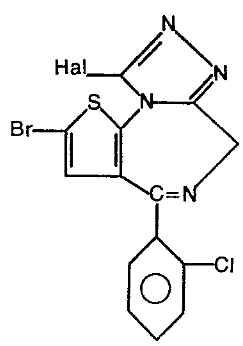

(II)

dans laquelle hal signifie un atome d'halogène avec un pipérazine de formule générale

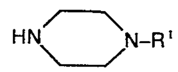

(III)

dans laquelle $R^1$ a la signification donnée plus haut, ou que

b) l'on déshydrogène de façon connue une composition de formule générale

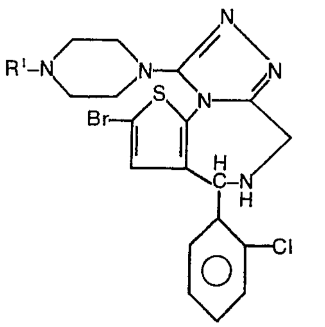

(IV)

dans laquelle $R^1$ a les significations données plus haut de façon connue en soi, ou que l'on fait réagir

c) le 8-bromo-6-(o-chlorophényl)-1-pipérazinyl-4H-s-triazolo[3,4c]thiéno[2,3e]1,4-diazépine avec l'oxyde d'éthylene pour obtenir le composé 1-(2-hydroxyéthyl)-pipérazinyl correspondant et eventuellement que l'on transforme le produit final ainsi obtenu de façon habituelle en un sel d'addition avec un acide physiologiquement compatible.

5. Préparation pharmaceutiques, caractérisées en ce qu'elles contiennent comme substances actives des composés de la formule générale I selon la revendication 1, eventuellement sous forme de leur sel d'addition avec des acides, en combinaison avec les adjuvants et/ou les supports habituels.